# EUROPEAN PATENT SPECIFICATION

(11) **EP 3 679 033 B1**
(45) Date of publication and mention of the grant of the patent: **21.10.2020**
(21) Application number: 18718091.4
(22) Date of filing: 28.03.2018
(51) Int. Cl.: C07D 409/14

(54) **METHOD OF SYNTHESIS OF 5,6-BIS(5-ALKOXYTHIOPHEN-2-YL)PYRAZINE-2,3-DICARBODINITRILES, DERIVATIVES OF DICYANOPYRAZINE AND USE THEREOF**
VERFAHREN ZUR SYNTHESE VON 5,6-BIS(5-ALKOXYTHIOPHEN-2-YL)PYRAZIN-2,3-DICARBODINITRIL, DERIVATEN VON DICYANOPYRAZIN UND DEREN VERWENDUNG
PROCÉDÉ DE SYNTHÈSE DE 5,6-BIS(5-ALCOXYTHIOPHÈN-2-YL)PYRAZINE-2,3-DICARBONITRILES, DÉRIVÉS DE DICYANOPYRAZINE ET UTILISATION ASSOCIÉE

(43) Date of publication of application: 15.07.2020
(73) Proprietor: Univerzita Pardubice, 532 10 Pardubice (CZ)
(72) Inventor: Bures, Filip, 530 09 Pardubice (CZ); Klikar, Milan, 512 51 Lomnice Nad Popelkou (CZ); Hlouskova, Zuzana, 530 09 Pardubice (CZ)
(74) Representative: Hartvichova, Katerina
(86) International application number: PCT/CZ2018/050014
(87) International publication number: WO 2019/185068

(56) References cited:
- Y. ZHAO; C. ZHANG; K. F. CHIN; O. PYTELA; G. WEI; H. LIU; F. BURES; Z. JIANG: "Dicyanopyrazine-derived push-pull chromophores for highly efficient photoredox catalysis", RSC. ADV., vol. 4, 30 June 2014 (2014-06-30), pages 30062-30067, XP002786607, DOI: 10.1039/c4ra05525j cited in the application
- X. LIU; X. YE; F. BURES; H. LIU; Z. JIANG: "Controllable Chemoselectivity in Visible-Light Photoredox Catalysis: Four Diverse Aerobic Radical Cascade Reactions", ANGEW. CHEM. INT. ED., vol. 54, 16 July 2015 (2015-07-16), pages 11443-11447, XP002786608, DOI: 10.1002/anie.201505193 cited in the application
- WEI, G.; ZHANG, C.; BURES, F.; YE, X.; TAN, C. AND JIANG, Z.: "Enantioselective Aerobic Oxidative C(sp3)-H Olefination of Amines via Cooperative Photoredox and Asymmetric Catalysis", ACS CATAL., vol. 6, 9 May 2016 (2016-05-09), pages 3708-3712, XP002786612, DOI: 10.1021/acscatal.6b00846

## Description

### Field of Art

The invention relates to a new and straightforward synthetic pathway towards photoredox catalysts based on pyrazine-2,3-dicarbonitrile scaffold.

### Background Art

Nowadays, a transformation of solar energy into the energy of chemical bonds represents very attractive and burgeoning area of photochemistry. This process, inspired by action of natural chlorophyll in photosynthesis, can be in a chemical laboratory enabled by a photoredox catalyst. Historically most abundant and well explored are metal complexes, especially ruthenium and iridium polypyridil complexes. These substances are well suited for visible light-induced photoredox reactions and their catalytic activities in various chemical transformations have been well documented. However, the current research trends in photoredox catalysis are clearly directed towards application of purely organic (metal-free) substances. Their application has in particular the following advantages: i) readily availability, ii) significantly reduced cost, iii) prevented contamination of the desired product with toxic heavy metals, iv) facile property tuning, and v) low catalyst loading. The most widely employed organic pigments involve xanthene dyes such as eosin Y, fluorescein, eosin B, and rose bengal but their further structural tuning is limited. Hence, a development of new and tunable organic substances with perspective photoredox catalytic activity is of high demand. The current portfolio of synthetic organic photoredox catalysts consists mainly of cyanoarenes, aromatic ketones as well as heteroaromatic substances such as flavins, various pyridinium salts, and perylene diimide.

A literature search of patents revealed, that iridium and ruthenium complexes belong to a prominent group of organometallic photoredox catalysts that were employed in light controllable radical polymerization, decarboxylative arylation or acylation, and fluoroalkylation of organic compounds. On the contrary, only few metal-free organic catalysts such as perylene, phenothiazine, phenoxazine, coronene, and acridinium salt and their utilization in organocatalyzed atom transfer radical polymerization or in the synthesis of substituted heteroarenes via direct C-H functionalization were patented so far.

In 2014, we have reported on push-pull chromophore based on pyrazine-2,3-dicarbonitrile bearing two 5-methoxythiophen-2-yl electron donors [Y. Zhao, C. Zhang, K. F. Chin, O. Pytela, G. Wei, H. Liu, F. Bureš, Z. Jiang, RSC. Adv. 2014, 4, 30062-30067]. This organic substance proved to be highly efficient in various visible-light induced photoredox reactions such as cross-dehydrogenative coupling (CDC), oxidation, oxidative hydroxylation, reductive dehalogenation, cascade reactions, pH-controlled photooxygenation of indoles, and enantioselective oxidative olefination of amines [X. Liu, X. Ye, F. Bureš, H. Liu, Z. Jiang, Angew. Chem. Int. Ed. 2015, 54, 11443-11447; C. Zhang, S. Li, F. Bureš, R. Lee, X. Ye, Z. Jiang, ACS Catal. 2016, 6, 6853-6860; G. Wei, C. Zhang, F. Bureš, X. Ye, C.-H. Tan, Z. Jiang, ACS Catal. 2016, 6, 3708-3712]. However, its synthesis shown below is either very low yielding or utilizes expensive, unstable, unavailable, and toxic starting compounds or catalytic system. Synthetic pathways leading towards 5,6-bis(5-methoxythiophen-2-yl)pyrazine-2,3-dicarbonitrile are depicted in Scheme 1.

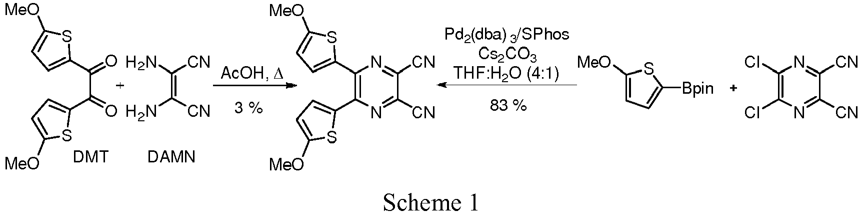

The original protocol towards 5,6-bis(5-methoxythiophen-2-yl)pyrazine-2,3-dicarbonitrile involved a separate preparation of 5,5'-dimethoxythenil (DMT, Scheme 1) and its subsequent acetic acid-catalyzed, low-yielding condensation with diaminomaleonitrile (DAMN) [Y. Zhao, C. Zhang, K. F. Chin, O. Pytela, G. Wei, H. Liu, F. Bureš, Z. Jiang, RSC. Adv. 2014, 4, 30062-30067]. A cross-coupling reaction utilizing unstable boronic acid ester and expensive catalytic system is an option [Y. Zhao, C. Zhang, K. F. Chin, O. Pytela, G. Wei, H. Liu, F. Bureš, Z. Jiang, RSC. Adv. 2014, 4, 30062-30067]. A new synthetic route, yielding high amounts of the above mentioned photoredox catalyst using less expensive, less toxic, more available, and highly stable reactants is therefore highly desirable.

### Disclosure of the Invention

The present invention relates to a new straightforward synthetic route to pyrazine-2,3-dicarbonitrile derivatives of general formula (I), bearing 5-alkoxythiophen-2-yl substituents attached at positions 5 and 6, and their utilization as catalysts in photoredox reactions. The main advantages of the invention are: i) possibility of one-pot synthetic protocol with a short reaction time, ii) inexpensive and readily available starting materials, iii) facile purification of the products (e.g. crystallization), iv) multigram synthesis, v) high catalytic efficiencies of the catalyst in the CDC reaction with diminished influence of the appended alkyl substituents R.

The subject of the present invention is a synthetic method of compounds of general formula (I) 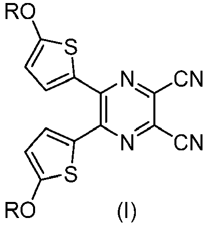 wherein R is linear or branched (C1-C8) alkyl, (C5-C6) cycloalkyl or benzyl, the method comprising the following steps:
step a) Friedel-Crafts reaction of precursor of general formula (II) 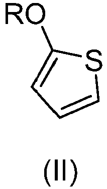 wherein R is as defined above,
   with oxalyldichloride, resulting in formation of a compound of general formula (III), 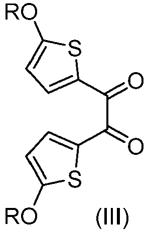 wherein R is as defined above,
   followed by
step b) Lewis acid catalyzed condensation reaction of the compound of general formula (III) with diaminomaleonitrile (DAMN) resulting in formation of the compound of general formula (I). The synthetic pathway of the compound of general formula (I) according to the present invention is depicted in Scheme 2.

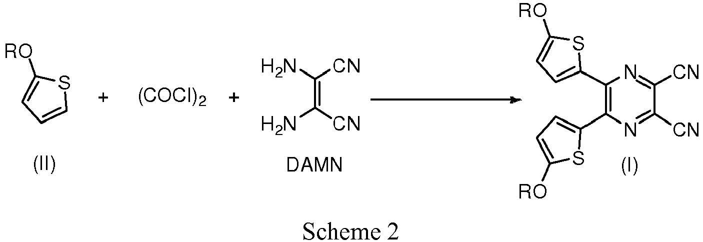

The synthesis according to the present invention starts from thiophene derivatives of general formula (II) and provides the desired pyrazine-2,3-dicarbonitrile derivatives of general formula (I) via a two-step procedure (the overall procedure is depicted in Scheme 2). The first step involves Friedel-Crafts reaction between the corresponding thiophene derivative of general formula (II) and oxalyldichloride. According to the current state-of-the-art, alkoxythiophenes were not applied as starting materials for twofold Friedel-Crafts reaction with oxalyldichloride so far.

In contrast to the original acid-catalyzed reaction, the subsequent condensation of the in-situ generated compounds of general formula (III) is carried out under Lewis acid catalysis. DAMN is well known electron poor 1,2-diamine capable to condense with 1,2-dicarbonyl compounds. However, its condensation with electron rich carbonyl compounds (such as compounds of general formula (III)), turned out to be very tedious and low-yielding.

According to the current state-of-the-art, DAMN can successfully be condensed with aldehydes, 1,2- and 1,3-dicarbonyl compounds under various conditions. The most widely employed is acid media represented by acetic acid and elevated temperature. Water, alcohols, 1,4-dioxane, benzene, and acetonitrile can further be used as reaction solvents. Beside acetic acid, the reaction can also be catalyzed by Bronsted acids such as HCl, H₂SO₄, trifluoroacetic acid, oxalic acid, and *p*-toluenesulfonic acid.

Starting thiophene derivatives of general formula (II) can be prepared from commercially available 2-methoxythiophene and the corresponding alcohol via modified procedure utilizing *p*-TsOH catalysis (Scheme 3) [N. Agarwal, C.-H. Hung, M. Ravikanth, Tetrahedron 2004, 60, 10671].

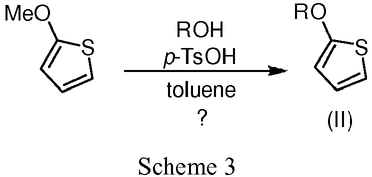

In one preferred embodiment of the method according to the present invention, the compound of general formula (III) formed in step a) is directly *in-situ* used in the following step b). Both steps are therefore performed in a one-pot reaction, which facilitates the synthesis, improves the time needed for the method, results in better yields of the final product (eliminates loss of the intermediate product of general formula (III) resulting from its isolation and purification), and therefore enabling economically more favorable overall procedure.

In one embodiment of the present invention, the catalyst for the Friedel-Crafts reaction in step a) is selected from a group comprising AlCl₃, SnCl₄, FeCl₃, BF₃Et₂O, TiCl₄, more preferably the catalyst for the Friedel-Crafts reaction is AlCl₃, SnCl₄ and/or TiCl₄, most preferably TiCl₄.

In one embodiment of the present invention, the Lewis acid catalyst in step b) is selected from a group comprising AlCl₃, SnCl₄, FeCl₃, BF₃·Et₂O, TiCl₄, more preferably the Lewis acid catalyst is BF₃·Et₂O and/or TiCl₄, most preferably TiCl₄.

In one preferred embodiment, the catalyst for both reaction steps a) and b) is TiCl₄.

In one embodiment of the present invention, the reaction steps a) and b) are carried out in a solvent selected from a group comprising tetrahydrofuran, ethyl acetate, toluene, 1,2-dichloroethane, more preferably the solvent is 1,2-dichloroethane.

In one preferred embodiment of the present invention, the step a) is carried out at temperature within the range of -10 to -20 °C, more preferably at about -15 °C.

In one preferred embodiment of the present invention, the step b) is carried out at temperature within the range of 60 to 120 °C, preferably within the range of 80 to 90 °C. Performing the reaction step b) at the temperature of boiling point of the solvent accelerates the reaction time. The optimized reaction time for the reaction between thiophene derivatives of general formula (II) and oxalyldichloride is about 20 min; a longer reaction times did not improve the yield. The subsequent condensation with DAMN is at least about 30 min.

### Compounds of general formula (A)

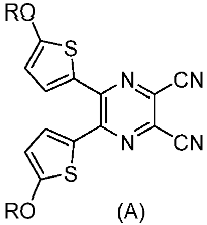

wherein R is linear or branched (C2-C8) alkyl, (C5-C6) cykloalkyl or benzyl,
with the proviso that R is not methyl may be useed as a catalyst for photoredox reactions. Preferably, the reactions are selected from those utilizing photoinduced electron transfer or energy transfer.

### Examples

The invention can be documented by the following experiments.

### Example 1: General method for preparation of precursors of general formula (II)

Starting thiophene derivatives of general formula (II) were prepared from commercially available 2-methoxythiophene (IIa) and the corresponding alcohol via modified procedure utilizing *p*-TsOH catalysis. Preparation of 2-alkoxythiophene derivatives IIb-e is depicted in Scheme 4 [N. Agarwal, C.-H. Hung, M. Ravikanth, Tetrahedron 2004, 60, 10671]. The attained yields of IIb-e range from 28 to 38 %.

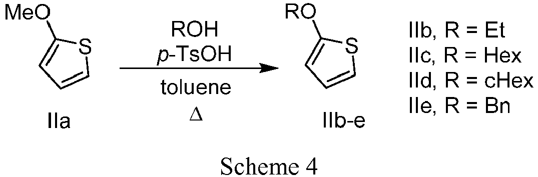

In a 100 mL dry autoclave, commercially available 2-methoxythiophene IIa (10 mmol) and corresponding alcohol (25 mmol) were dissolved in dry toluene (50 mL). *p-*Toluenesulfonic acid (1 mmol) was added and the reaction mixture was stirred at 90 °C for 24 h. The solvent was partially evaporated in vacuo and the residue was purified by flash chromatography using a mixture of DCM/Hex 1:1 as eluent.

### 2-Ethoxythiophene (IIb)

The title compound was synthesized from IIa (1.14 g; 10 mmol) and ethanol (1.15 g; 25 mmol) following the general method. Yield: 384 mg (30 %); colorless liquid. *R*_{f} = 0.95 (SiO₂; Hex/DCM 1:1). EI/MS (70 eV): *m*/*z* 128 (M⁺, 45 %), 100 (100), 84 (18), 71 (20), 55 (15), 45 (15). ¹H NMR (CDCl₃, 400 MHz, 25 °C): *δ* = 1.41 (t, *J* = 7.2 Hz, 3H, CH₃), 4.08 (q, *J* = 7.2 Hz, 2H, CH₂), 6.19 (dd, *J₁* = 4 Hz, *J₂* = 1.2 Hz, 1H, CHₜₕ), 6.53 (dd, *J₁* = 6 Hz, *J₂* = 1.2 Hz, 1H, CHₜₕ), 6.70 ppm (dd, *J₁* = 6 Hz, *J₂* = 4 Hz, 1H, CHₜₕ). Other analytic data were in accordance with the literature data [Y. Guo, X.-M. Fan, M. Nie, H.-W. Liu, D.-H. Liao, X.-D. Pan, Y.-F. Ji, Eur. J. Org. Chem. 2015, 4744].

### 2-Hexyloxythiophene (IIc)

The title compound was synthesized from IIa (1.14 g; 10 mmol) and hexan-1-ol (2.55 g; 25 mmol) following the general method. Yield: 700 mg (38 %); colorless liquid. *R*_{f} = 0.95 (SiO₂; Hex/DCM 1:1). EI/MS (70 eV): *m*/*z* 184 (M⁺, 12 %), 100 (100), 55 (8), 45 (25). ¹H NMR (CDCl₃, 400 MHz, 25 °C): *δ* = 0.94 (t, *J* = 7.2 Hz, 3H, CH₃), 1.31-1.34 (m, 4H, CH₂), 1.41-1.47 (m, 2H, CH₂), 1.73-1.80 (m, 2H, CH₂), 4.01 (t, *J* = 6.8 Hz, 2H, CH₂), 6.18 (dd, *J₁* = 4 Hz, *J₂* = 1.2 Hz, 1H, CHₜₕ), 6.52 (dd, *J₁* = 6 Hz, *J₂* = 1.2 Hz, 1H, CHₜₕ), 6.70 ppm (dd, *J₇* = 6 Hz, *J₂* = 4 Hz, 1H, CHₜₕ). ¹³C NMR (CDCl₃, 100 MHz, 25 °C): *δ* = 14.2, 22.7, 25.6, 29.2, 31.6, 74.6, 107.1, 113.2, 124.6, 138.9 ppm. These data complete the only boiling point of IIc (66 °C/0.26 mbar) reported in the literature [P. Wegener, M. Feldhues, H. Litterer, Hoechst Aktiengesellschaft - US4931568, 1990, A1].

### 2-Cyclohexyloxythiophene (IId)

The title compound was synthesized from IIa (1.14 g; 10 mmol) and cyclohexanol (2.5 g; 25 mmol) following the general method. Yield: 637 mg (35 %); colorless liquid. *R*_{f} = 0.95 (SiO₂; Hex/DCM 1:1). EI/MS (70 eV): *m*/*z* 182 (M⁺, 5 %), 100 (100), 83 (9), 55 (29). ¹H NMR (CDCl₃, 400 MHz, 25 °C): *δ* = 1.28-1.38 (m, 4H, CH₂), 1.52-1.60 (m, 4H, CH₂), 1.97-2.01 (m, 2H, CH₂), 4.03-4.09 (m, 1H, CH), 6.25 (dd, *J₁* = 4 Hz, *J₂* = 1.2 Hz, 1H, CHₜₕ), 6.56 (dd, *J₁* = 6 Hz, *J₂* = 1.2 Hz, 1H, CHₜₕ), 6.70 ppm (dd, *J₁* = 6 Hz, *J₂* = 4 Hz, 1H, CHₜₕ). ¹³C NMR (CDCl₃, 100 MHz, 25 °C): *δ* = 23.8, 25.7, 31.9, 82.9, 107.5, 112.8, 124.8, 139.9 ppm. The analytical data were in accordance with the literature data [K. Morimitsu, K. Shibata, S. Kobatake, M. Irie, J. Org. Chem. 2002, 67, 4574].

### 2-Benzyloxythiophene (IIe)

The title compound was synthesized from IIa (1.14 g; 10 mmol) and benzyl alcohol (2.7 g; 25 mmol) following the general method. Yield: 532 mg (28 %); colorless liquid. R_{f} = 0.95 (SiO₂; Hex/DCM 1:1). EI/MS (70 eV): *m*/*z* 190 (M⁺, 6 %), 91 (100), 65 (8). ¹H NMR (CDCl₃, 400 MHz, 25 °C): δ = 5.09 (s, 2H, CH₂), 6.29 (dd, *J₁* = 4 Hz, *J₂* = 1.2 Hz, 1H, CHₜₕ), 6.58 (dd, *J₁* = 6 Hz, *J₂* = 1.2 Hz, 1H, CHₜₕ), 6.73 (dd, *J₁* = 6 Hz, *J₂* = 4 Hz, 1H, CHₜₕ), 7.36-7.46 ppm (m, 5H, CHₚₕ). ¹³C NMR (CDCl₃, 100 MHz, 25 °C): δ = 75.8, 105.8, 112.5, 124.8, 128.1, 128.6, 128.8, 136.2, 165.4 ppm. The analytical data were in accordance with the literature data [K. Nakamoto, M. Matsukura, K. Tanaka, S. Inoue, I. Tsukada, T. Haneda, N. Ueda, S. Abe, K. Sagane, Eisai R&D Management Co., Ltd. - EP1782811, 2007, A1].

### Example 2: General method for preparation of compound of general formula (I)

TiCl₄ (5 mmol) was added dropwise to 1,2-dichlorethane (DCE, 7.5 mL) at -15 °C. A solution of oxalyldichloride (2.5 mmol) in DCE (1.5 mL) was added dropwise and the resulting mixture was stirred for 5 min. A mixture of a compound of general formula (II) (5 mmol) and pyridine (5.5 mmol) in DCE (2.5 mL) was subsequently added and the reaction mixture was stirred for 20 min at -15 °C. Diaminomaleonitrile (DAMN, 5.5 mmol) was added and the reaction mixture was heated at reflux for 30 minutes. The reaction mixture was filtrated via a short pad of silica gel using DCM as auxiliary eluent. The filtrate was evaporated and the crude product was crystallized from toluene.

### 5,6-Bis(5-methoxythiophen-2-yl)pyrazine-2,3-dicarbonitrile (Ia)

The title compound was synthesized from IIa (570 mg; 5 mmol), oxalyldichloride (318 mg; 2.5 mmol), TiCl₄ (950 mg; 5 mmol), pyridine (435 mg; 5.5 mmol) and DAMN (594 mg; 5.5 mmol) following the general method. Yield: 558 mg (63 %); orange solid. *R*_{f} = 0.85 (SiO₂; DCM); mp = 177-179 °C. ¹H NMR (CDCl₃, 500 MHz, 25 °C): *δ* = 3.99 (s, 6H, CH₃), 6.18 (d, *J* = 4.4 Hz, 2H, CH), 7.67 ppm (d, *J* = 4.4 Hz, 2H, CH). ¹³C NMR (CDCl₃, 125 MHz, 25 °C): *δ* = 60.8, 106.3, 113.7, 124.7, 126.3, 131.2, 146.7, 173.9 ppm. HR-MALDI-MS (DHB): calcd for C₁₆H₁₁N₄O₂S₂ ([M+H]⁺) 355.0318, found 355.0317. The analytical data were in accordance with the literature data [Y. Zhao, C. Zhang, K. F. Chin, O. Pytela, G. Wei, H. Liu, F. Bureš, Z. Jiang, RSC. Adv. 2014, 4, 30062-30067].

### 5,6-Bis(5-ethoxythiophen-2-yl)pyrazine-2,3-dicarbonitrile (Ib)

The title compound was synthesized from IIb (640 mg; 5 mmol), oxalyldichloride (318 mg; 2.5 mmol), TiCl₄ (950 mg; 5 mmol), pyridine (435 mg; 5.5 mmol) and DAMN (594 mg; 5.5 mmol) following the general method. Yield: 554 mg (58 %); orange solid. *R*_{f} = 0.85 (SiO₂; DCM); mp = 170-172 °C. ¹H NMR (CDCl₃, 400 MHz, 25 °C): *δ* = 1.47 (t, *J* = 7.2 Hz, 6H, CH₃), 4.20 (q, *J* = 7.2 Hz, 4H, CH₂), 6.17 (d, *J* = 4.4 Hz, 2H, CH), 7.67 ppm (d, *J* = 4.4 Hz, 2H, CH). ¹³C NMR (CDCl₃, 100 MHz, 25 °C): *δ* = 14.8, 70.2, 106.8, 113.7, 124.5, 126.2, 131.3, 146.8, 173.0 ppm. HR-MALDI-MS (DHB): calcd for C₁gH₁₄N₄O₂S₂ ([M]⁺) 382.05527, found 382.05592.

### 5,6-Bis(5-hexyloxythiophen-2-yl)pyrazine-2,3-dicarbonitrile (Ic)

The title compound was synthesized from IIc (920 mg; 5 mmol), oxalyldichloride (318 mg; 2.5 mmol), TiCl₄ (950 mg; 5 mmol), pyridine (435 mg; 5.5 mmol) and DAMN (594 mg; 5.5 mmol) following the general method. Yield: 618 mg (50 %); orange solid. *R*_{f} = 0.9 (SiO₂; DCM); mp = 126-130 °C. ¹H NMR (CDCl₃, 500 MHz, 25 °C): *δ* = 0.89-0.92 (m, 6H, CH₃), 1.33-1.35 (m, 8H, CH₂), 1.44-1.47 (m, 4H, CH₂), 1.79-1.85 (m, 4H, CH₂), 4.13 (t, *J =* 6.5 Hz, 4H, CH₂), 6.16 (d, *J* = 4.4 Hz, 2H, CH), 7.67 ppm (d, *J* = 4.4 Hz, 2H, CH). ¹³C NMR (CDCl₃, 125 MHz, 25 °C): *δ* = 14.3, 22.8, 25.8, 29.3, 31.7, 74.8, 106.9, 113.8, 124.5, 126.3, 131.4, 146.9, 173.5 ppm. HR-MALDI-MS (DHB): calcd for C₂₆H₃₁N₄O₂S₂ ([M+H]⁺) 495.18047, found 495.18839.

### 5,6-Bis(5-cyclohexyloxythiophen-2-yl)pyrazine-2,3-dicarbonitrile (Id)

The title compound was synthesized from IId (910 mg; 5 mmol), oxalyldichloride (318 mg; 2.5 mmol), TiCl₄ (950 mg; 5 mmol), pyridine (435 mg; 5.5 mmol) and DAMN (594 mg; 5.5 mmol) following the general method. Yield: 515 mg (42 %); orange solid. *R*_{f} = 0.9 (SiO₂; DCM); mp = 125-129 °C. ¹H NMR (CDCl₃, 400 MHz, 25 °C): *δ* = 1.31-1.44 (m, 6H, CH₂), 1.54-1.67 (m, 6H, CH₂), 1.80-1.83 (m, 4H, CH₂), 2.03-2.07 (m, 4H, CH₂), 4.22-4.28 (m, 2H, CH), 6.17 (d, *J* = 4.4 Hz, 2H, CH), 7.68 ppm (d, *J* = 4.4 Hz, 2H, CH). ¹³C NMR (CDCl₃, 100 MHz, 25 °C): *δ* = 23.7, 25.4, 31.7, 83.78, 108.3, 113.8, 124.5, 126.1, 131.3, 146.7, 172.3 ppm. HR-MALDI-MS (DHB): calcd for C₂₆H₂₇N₄O₂S₂ ([M+H]⁺) 491.15700, found 491.15771.

### 5,6-Bis(5-benzyloxythiophen-2-yl)pyrazine-2,3-dicarbonitrile (Ie)

The title compound was synthesized from IIe (950 mg; 5 mmol), oxalyldichloride (318 mg; 2.5 mmol), TiCl₄ (950 mg; 5 mmol), pyridine (435 mg; 5.5 mmol) and DAMN (594 mg; 5.5 mmol) following the general method. Yield: 120 mg (10 %); red solid. *R*_{f} = 0.85 (SiO₂; DCM); mp = 140 °C (decomp.). ¹H NMR (CDCl₃, 400 MHz, 25 °C): *δ* = 5.18 (s, 4H, CH₂), 6.25 (d, *J* = 4.4 Hz, 2H, CH), 7.38-7.43 (m, 10H, CH), 7.65 ppm (d, *J* = 4.4 Hz, 2H, CHₜₕ). ¹³C NMR (CDCl₃, 100 MHz, 25 °C): *δ* = 76.0, 107.7, 113.6, 124.9, 126.4, 128.2, 129.0, 129.1, 131.2, 134.9, 146.7, 172.4 ppm. HR-MALDI-MS (DHB): calcd for C₂₈H₁₉N₄O₂S₂ ([M+H]⁺) 507.09440, found 507.09592.

### Example 3: Testing of catalytic performance - general method for the cross-dehydrogenative coupling (CDC) reaction

The catalytic performance of all prepared compounds of general formula (I) has been screened in benchmark cross-dehydrogenative coupling reaction between *N*-phenyltetrahydroisoquinoline IV (THIQ) and nitromethane providing product V, according to the reaction Scheme 5.

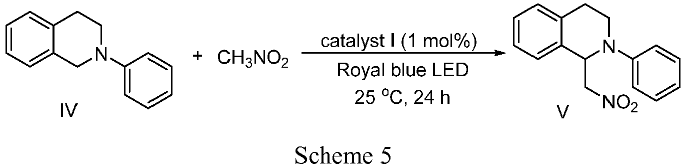

In a 10 mL snap vial equipped with a magnetic stirring bar, *N*-phenyltetrahydroisoquinoline IV (THIQ, 31 mg, 0.15 mmol) was dissolved into nitromethane (1.5 mL) and then photocatalyst I (1.5 µmol) was added. The reaction mixture was stirred under irradiation by a Royal Blue LED at 25 °C (temperature was maintained in an incubator) from 5 cm distance. After 24 hours, the solvent was removed *in vacuo* and the whole residue was analyzed by ¹H NMR (CDCl₃) to estimate the conversion and subsequently purified by flash chromatography (SiO₂; petroleum ether (PE):ethyl acetate (EA) = 10:1) to obtain pure product V.

The obtained results are listed in Table 1.

**Table 1. Benchmark CDC reaction.**

| Catalyst (R) | Conversion (%)^{a} | Isolated yield (%) |
|---|---|---|
| Ia (Me) | 100 | 50 |
| Ib (Et) | 100 | 45 |
| Ic (Hex) | 100 | 50 |
| Id (cHex) | 100 | 60 |
| Ie (Bn) | 100 | 38 |

| | | |
|---|---|---|
| ^{a}Estimated from ¹H NMR spectra of the crude reaction mixtures. | | |

As can be seen from the data in Table 1, all catalysts were able to convert IV to V within 24 h under irradiation with the blue LED (447.5 nm). The isolated yields range from 38 to 60 %. Except the catalysts Ie bearing benzyl R-substituent, the isolated yields (45-60 %) can be considered as unaltered by the alkoxy residues appended to the catalyst.

## Claims

1. A method of synthesis of compounds of general formula (I) 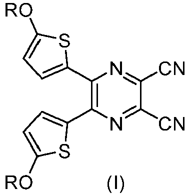 wherein R is linear or branched (C1-C8) alkyl, (C5-C6) cycloalkyl or benzyl, the method comprising the following steps:
step a) Friedel-Crafts reaction of precursor of general formula (II) 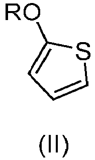 wherein R is as defined above,
with oxalyldichloride, resulting in formation of a compound of general formula (III), 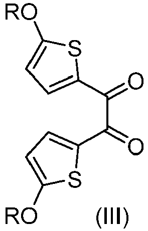 wherein R is as defined above,
followed by
step b) Lewis acid catalyzed condensation reaction of the compound of general formula (III) with diaminomaleonitrile (DAMN) resulting in formation of the compound of general formula (I).

2. The method according to claim 1, **characterized in that** the compound of general formula (III) formed in step a) is directly *in-situ* used in the following step b).

3. The method according to claim 1, **characterized in that** the catalyst for the Friedel-Crafts reaction in step a) is selected from a group comprising AlCl₃, SnCl₄, FeCl₃, BF₃Et₂O, TiCl₄, preferably the catalyst for the Friedel-Crafts reaction is AlCl₃, SnCl₄ and/or TiCl₄, more preferably the catalyst for the Friedel-Crafts reaction is TiCl₄.

4. The method according to claim 1, **characterized in that** the Lewis acid catalyst in step b) is selected from a group comprising AlCl₃, SnCl₄, FeCl₃, BF₃·Et₂O, TiCl₄, preferably the Lewis acid catalyst is BF₃·Et₂O and/or TiCl₄, more preferably the Lewis acid catalyst is TiCl₄.

5. The method according to claim 2, **characterized in that** the catalyst for both reaction steps a) and b) is TiCl₄.

6. The method according to any one of the preceding claims, **characterized in that** the reaction steps a) and b) are carried out in a solvent selected from a group comprising tetrahydrofuran, ethyl acetate, toluene, 1,2-dichloroethane, preferably the solvent is 1,2-dichloroethane.

7. The method according to any one of the preceding claims, **characterized in that** the step a) is carried out at temperature within the range of -10 to -20 °C.

8. The method according to any one of the preceding claims, **characterized in that** the step b) is carried out at temperature within the range of 60 to 120 °C, preferably within the range of 80 to 90 °C.

## Patentansprüche

1. Ein Verfahren zur Synthese von Verbindungen der allgemeinen Formel (I) 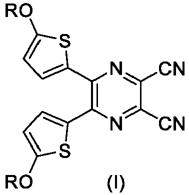 worin R lineares oder verzweigtes (C1-C8)-Alkyl, (C5-C6)-Cycloalkyl oder Benzyl ist, wobei das Verfahren umfasst die folgenden Schritte:
Schritt a) Friedel-Crafts-Reaktion einer Ausgangsverbindung der allgemeinen Formel (II) 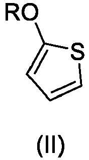 worin R ist wie oben definiert,
mit Oxalyldichlorid, was zur Bildung einer Verbindung der allgemeinen Formel (III) führt, 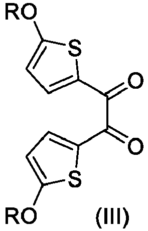 worin R ist wie oben definiert,
gefolgt von
Schritt b) Lewis-Säure-katalysierte Kondensationsreaktion der Verbindung der allgemeinen Formel (III) mit Diaminomaleonitril (DAMN), die zur Bildung der Verbindung der allgemeinen Formel (I) führt.

2. Verfahren nach Anspruch 1, **dadurch gekennzeichnet, dass** die in Schritt a) gebildete Verbindung der allgemeinen Formel (III) in dem folgenden Schritt b) direkt *in situ* verwendet wird.

3. Verfahren nach Anspruch 1, **dadurch gekennzeichnet, dass** der Katalysator für die Friedel-Crafts-Reaktion in Schritt a) ausgewählt ist aus einer Gruppe bestehend aus AlCl₃, SnCl₄, FeCl₃, BF₃·Et₂O, TiCl₄, vorzugsweise der Katalysator für die Friedel-Crafts-Reaktion AlCl₃, SnCl₄ und/oder TiCl₄ ist, bevorzugter der Katalysator für die Friedel-Crafts-Reaktion ist TiCl₄.

4. Verfahren nach Anspruch 1, **dadurch gekennzeichnet, dass** der Lewis-Säure-Katalysator in Schritt b) ausgewählt ist aus einer Gruppe, umfassend AlCl₃, SnCl₄, FeCl₃, BF₃·Et₂O, TiCl₄, vorzugsweise ist der Lewis-Säure-Katalysator BF₃·Et₂O und/oder TiCl₄; bevorzugter ist der Lewis-Säure-Katalysator TiCl₄.

5. Verfahren nach Anspruch 2, **dadurch gekennzeichnet, dass** der Katalysator für beide Reaktionsschritte a) und b) TiCl₄ ist.

6. Verfahren nach einem der vorhergehenden Ansprüche, **dadurch gekennzeichnet, dass** die Reaktionsschritte a) und b) in einem Lösungsmittel ausgewählt aus einer Gruppe, umfassend Tetrahydrofuran, Ethylacetat, Toluol, 1,2-Dichlorethan, durchgeführt werden; vorzugsweise ist das Lösungsmittel 1,2-Dichlorethan.

7. Verfahren nach einem der vorhergehenden Ansprüche, **dadurch gekennzeichnet, dass** der Schritt a) bei einer Temperatur im Bereich von -10 bis -20 °C durchgeführt wird.

8. Verfahren nach einem der vorhergehenden Ansprüche, **dadurch gekennzeichnet, dass** der Schritt b) bei einer Temperatur im Bereich von 60 bis 120 °C, vorzugsweise im Bereich von 80 bis 90 °C durchgeführt wird.

## Revendications

1. Un procédé de synthèse de composés de formule générale (I) 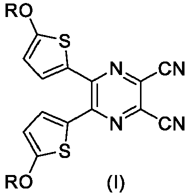 où R est un (C1-C8) alkyle linéaire ou ramifié, (C5-C6) cycloalkyle ou benzyle, le procédé comprenant les étapes suivantes:
une étape a) de réaction de Friedel-Crafts du précurseur de formule générale (II) 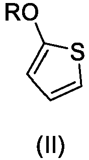 où R est tel que défini ci-dessus,
avec un oxalyldichlorure, entraînant la formation d'un composé de formule générale (III), 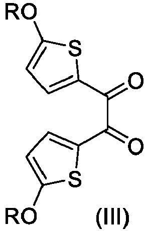 où R est tel que défini ci-dessus,
suivi par
une étape b) de réaction de condensation catalysée par l'acide de Lewis du composé de formule générale (III) avec le diaminomaleonitrile (DAMN) conduisant à la formation du composé de formule générale (I).

2. Le procédé selon la revendication 1, **caractérisé en ce que** le composé de formule générale (III) formé à l'étape a) est directement utilisé in-situ dans l'étape b) suivante.

3. Le procédé selon la revendication 1, **caractérisé en ce que** le catalyseur de la réaction de Friedel-Crafts à l'étape a) est choisi dans un groupe comprenant AlCl₃, SnCl₄, FeCl₃, BF₃·Et₂O, TiCl₄, de préférence le catalyseur de la réaction de Friedel-Crafts est AlCl₃, SnCl₄ et / ou TiCl₄, plus préférablement le catalyseur pour la réaction de Friedel-Crafts est TiCl₄.

4. Le procédé selon la revendication 1, **caractérisé en ce que** le catalyseur acide de Lewis de l'étape b) est choisi dans un groupe comprenant AlCl₃, SnCl₄, FeCl₃, BF₃·Et₂O, TiCl₄, de préférence le catalyseur acide de Lewis est BF₃·Et₂O et / ou TiCl₄, plus préférablement, le catalyseur acide de Lewis est TiCl₄.

5. Le procédé selon la revendication 2, **caractérisé en ce que** le catalyseur pour les deux étapes de réaction a) et b) est TiCl₄.

6. Le procédé selon l'une quelconque des revendications précédentes, **caractérisé en ce que** les étapes réactionnelles a) et b) sont effectuées dans un solvant choisi dans un groupe comprenant le tétrahydrofurane, l'acétate d'éthyle, le toluène, le 1,2-dichloroéthane, de préférence le solvant est 1,2 dichloroéthane.

7. Le procédé selon l'une quelconque des revendications précédentes, **caractérisé en ce que** l'étape a) est réalisée à une température dans la rangée de -10 à -20 °C.

8. Le procédé selon l'une quelconque des revendications précédentes, **caractérisé en ce que** l'étape b) est réalisée à une température dans la rangée de 60 à 120 °C, de préférence dans la rangée de 80 à 90 °C.
